# EUROPEAN PATENT APPLICATION

(11) **EP 3 872 584 A1**
(43) Date of publication of application: **01.09.2021**
(21) Application number: 20159958.6
(22) Date of filing: 28.02.2020
(51) Int. Cl.: G05B 13/02, G06K 9/62, G06K 9/66, G06N 3/00, G06N 3/02, G06N 5/00, G06N 7/00, G06N 20/00, G06N 20/20, G06T 7/00, G16H 50/20, G16H 50/30, A61B 6/00, A61B 6/03, G01R 33/56

(54) **TECHNIQUE FOR DETERMINING AN INDICATION OF A MEDICAL CONDITION**

(71) Applicant: Deepc GmbH, 81671 München (DE)
(72) Inventor: Pfister, Franz, 80538 München (DE)
(74) Representative: Laqua, Bernd Christian Kurt

(57) **Abstract**

A medical data processing technique for determining an indication of a medical condition is disclosed. A method implementation of the technique comprises selecting (202), based on at least one property associated with medical data of a test instance, at least one model out of a plurality of models, wherein each of the plurality of models is generated by a learning algorithm and configured to provide a model-specific indication of the medical condition, determining (204), using each of the at least one selected model, a respective model-specific indication, and determining (206), based on the model-specific indications, the indication of the medical condition.

## Description

### Technical Field

The present disclosure generally relates to the field of medical data processing. In particular, a technique for enabling a determination of an indication of a medical condition is presented. The technique may be embodied in methods, computer programs and apparatuses.

### Background

Computer-based techniques for medical analysis, including machine learning techniques, have advanced in recent years and are considered a powerful tool to support doctors in medical diagnosis. Research works support the claim that such techniques have the potential to significantly improve a physician's or a radiologist's workflow in terms of diagnostic quality (e.g., performance in spotting malicious lung nodules in chest computed tomography (CT) images can be improved) or in terms of time-efficiency (e.g., time to diagnosis can be reduced from minutes to seconds). Apart from medical imaging, workflows in other diagnostic pillars also hold the potential to be improved by these techniques, such as in electroencephalography (EGG) (e.g., detecting signs of Epilepsy in EEG signals) or on laboratory test results (e.g., predicting haematologic disease from blood test results), for example.

Existing solutions have proven to yield high predictive performance. However, they suffer from several drawbacks. For instance, some known approaches exhibit bad generalization. In particular, these approaches may not be applicable in practice, because they cannot be applied on types of data differing from the type of data used for training, which may occur with different recording modalities (e.g., CT images, magnetic resonance (MR) images, two-dimensional X-ray images or EEG signals) or data qualities (e.g., an image resolution or a sample rate of EEG signals), for example. In addition, several of the currently known approaches may exhibit low robustness, leading to big changes in determined predictions in case of only small changes in the input data. Still further, some known approaches may not be safe for use, as they are not capable of reflecting uncertainties in the determined predictions. Last but not least, deep learning models are generally not well explainable, as their output may be considered to be produced by a trained black box function and may as such not be well traceable by a human. This may yield low trust by users and complicates bug fixing.

### Summary

Accordingly, there is a need for a technical implementation which allows providing a reliable and flexible determination of an indication of a medical condition.

According to a first aspect, a medical data processing method for determining an indication of a medical condition is provided. The method comprises selecting, based on at least one property associated with medical data of a test instance, at least one model out of a plurality of models. Each of the plurality of models is generated by a learning algorithm and configured to provide a model-specific indication of the medical condition based on the medical data. The method further comprises determining, using each of the at least one selected model, a respective model-specific indication of the medical condition based on the medical data. The method also comprises determining, based on the model-specific indications, the indication of the medical condition.

The at least one model may be selected out of the plurality of models based on the at least one property, wherein the property may be related to the test instance. The selection may thus depend on the test instance at hand. The selection may also be referred to as dynamical selection. Such a dynamical selection may ensure a robust determination of the indication of the medical condition in dependence on the at least one property. By providing different models suitable for different properties of the medical data, the most suitable models may be selected depending on the medical data at hand. For example, if the at least one property is an image modality of a medical image comprised in the medical data of the test instance, only models which are capable of determining the indication based on such image modality may be selected. The at least one selected model may have been generated by a learning algorithm using training data of the same image modality as the medical data of the test instance. This may improve reliability of the determined indication.

More than one model may be selected from the plurality of models. Each of the selected models may then provide the respective model-specific indication and the (e.g., final) indication of the medical condition may be determined based on all these model-specific indications. For that reason, the (e.g., group of) selected models, each of which has been generated by a (e.g., different) learning algorithm, may be referred to as an ensemble. If the selected models are generated by machine learning algorithms, the (e.g., group of) selected models may be referred to as a machine learning ensemble. Outputs of different models may advantageously be aggregated or combined for determining the indication. As different models may yield different model-specific indications based on the same medical data, such an aggregation or combination may increase the overall reliability of the determined indication.

The test instance may be a dataset related to a patient, for example. The dataset may comprise the medical data and, optionally, data (e.g., metadata) describing or defining the at least one property associated with the medical data of the test instance. The patient may be referred to as a patient to be assigned a medical diagnosis or the indication of the medical condition. The medical data may in other words relate to the patient. The medical data may relate to the patient in that it describes medical properties of the patient. The medical data may comprise data related to (e.g., describing or representing) a measurement of at least one property of a body of the patient, wherein the measurement may be a medical measurement. The medical data may relate to (e.g., describe or represent) a state of the body of the patient, wherein the state may be an anatomical, physical or physiological state. The medical data may comprise data describing an EEG measurement, data describing a blood test result, data describing a medical image or the like. The medical data may be omics data such as genomic, proteomic, glycomic, immunomic, brainomic data or the like. The medical data may comprise or be a medical image (e.g., of the patient). The medical image may be acquired by medical image acquisition (e.g., of at least a part of the body of the patient), and may be a CT image, a MR image, an ultrasonic image, or a camera image, for example. The step of acquiring the medical image may be, but may not necessarily be, part of the method disclosed herein. The medical data may relate to the patient in that it comprises or consists of at least one medical image of the patient.

The medical condition may describe or represent a distinctiveness or a peculiar (e.g., anatomical, physical or physiological) property of a patient to which the test instance relates. The medical condition or the indication of the medical condition may be a (e.g., class or higher-level) label predicted or determined for the medical data of the test instance. The medical condition or the (e.g., class or higher-level) label may describe that a patient is healthy, that a patient is not healthy, that a patient has a certain (e.g., predetermined) symptom, that the patient exhibits a medical anomaly (e.g., compared to healthy patients), or the like.

The learning algorithm may be an algorithm that learns (or "performs an optimization") using labeled or unlabeled training data as an input, wherein the model may be the output of the learning algorithm. The learning algorithm may be an algorithm that is used to determine (or "optimize") parameters to be used in or by a model (e.g., a model of the plurality of models), wherein labeled or unlabeled training data may be used as an input to learn or determine the (e.g. optimal) parameters. The learning algorithm may define a sequence of particular method steps and may be used to optimize the parameters of a statistical model, for example. In other variants, the learning algorithm may be a machine learning algorithm that optimizes the parameters of a machine learning model. A least one of the plurality of models may be an adversarial autoencoder or a neural network, for example.

The property associated with the medical data of the test instance may be a property described by the metadata of the medical data (e.g., in a header of the medical image), or a property extracted from (e.g., contents comprised in) the medical data (e.g., the medical image) of the test instance, for example. The property associated with the medical data of the test instance may be specific for the test instance and/or the medical data of the test instance. For example, the at least one property associated with the medical data may comprise or be a feature (e.g., a slice thickness, an image resolution, an image contrast, a radiomics feature, an image modality, etc.) of the medical image. The at least one property associated with the medical data may comprise or be a characteristic (e.g., an age, a gender, an ethnicity, etc.) of the patient to which the medical data relates.

The step of selecting the at least one model may be performed by a selector, for example. The selector may comprise or consist of a selector model. The selector may represent or define steps of a part of the method described herein. The step of selecting the at least one model may be performed before (or "prior to") determining the model-specific indications. The model-specific indications may be determined only by the selected at least one model. In other words, the step of determining the respective model-specific indication of the medical condition based on the medical data may be performed using only each of the at least one selected model. This may decrease the amount of computing time and resources needed to determine the indication of the medical condition.

In one variant, the step of selecting may comprise comparing, individually for each of the plurality of models, the at least one property associated with the medical data of the test instance and at least one property associated with training data used for generating the individual model. This may ensure that (e.g., only) the most suitable models for determining the indication of the medical condition are selected from the plurality of models, which may result in a (e.g., well suited or optimal) ensemble of models dynamically selected based on metadata of the (e.g., medical data of the) test instance depending on the use case.

The training data may comprise or consist of medical data related to a plurality of different patients. The training data may comprise or consist of a plurality of medical images (e.g., of different patients), also referred to as training images. The training images may be acquired by medical image acquisition, e.g., CT images, MR images, ultrasonic images, camera images, or the like. The training data may comprise or consist of a different type of content, such as EEG signals, blood test results, genomics, or the like. The property associated with the training data may be a property described by metadata (e.g., in a header or a label) of the training data, or a property extracted from (e.g., contents comprised in) the training data (e.g., the training images). The property associated with the training data may be specific for the training data. For example, the at least one property associated with the training data may comprise or be a feature (e.g., a slice thickness, an image resolution, an image contrast, a radiomics feature, an image modality, etc.) of at least one training image, which training image may be comprised in or correspond to the training data. The at least one property associated with the training data may comprise or be a characteristic (e.g., an age, a gender, an ethnicity, etc.) of one or more patients to which the training data relates. In one variant, the at least one property associated with the medical data of the test instance and the at least one property associated with the training data used for generating the individual model may be of a same type of property.

The indication of the medical condition may be determined further based on at least one attribute chosen from a result of the comparing, empirical performances of each of the plurality of models and a degree of explainability of each of the plurality of models. The at least one attribute may be specific for each of the models, respectively. This may enable prioritizing one model over another, depending on the model-specific attributes, in order to determine the indication of the medical condition more reliably. For example, results obtained by models less suitable for determining the model-specific indication may be given less weight compared with models more suitable for determining the model-specific indication.

The result of the comparing may be a degree of suitability (e.g., expressed as a numerical or a binary value) of each of the plurality of models for determining a model-specific indication for the medical data of the test instance. The degree of suitability may be obtained from a database storing a plurality of degrees of suitability associated with one or more (e.g., possible, or ranges of) properties of medical data. The empirical performances of each of the plurality of models may be determined using predetermined, preferably labeled, medical data to determine a respective model-specific indication and measuring the performance of the respective model. The performance may be a temporal performance (e.g., a time it takes for a model to provide a model-specific indication using predetermined, limited computing resources), a resampling performance and/or a reliability performance (e.g., a probability or degree of false positive detection, a probability or degree of false negative detection, or the like). The empirical performances may be obtained from a database storing a plurality of empirical performances associated with one or more of the models.

The degree of explainability of a model may be a degree of comprehensibility or traceability by a human of the results output by the model. The higher the degree of explainability of the model, the better a user may be able to understand how a model-specific indication is determined using the model. The degree of explainability of a model may be a (e.g., numerical or binary) value associated with the model, which may be determined or defined manually. For example, a low degree of explainability may be determined for models relying on one or more neural networks as their output may be considered to be produced by a (trained) black box function, and a higher degree of explainability may be determined for models the output results of which are better traceable than for neural networks. The degree of explainability may be predetermined for one or more, preferably all, of the plurality of models. The degree of explainability may be obtained from a database, e.g., from a database storing a plurality of degrees of explainability associated with one or more of the plurality of models.

The indication of the medical condition may be determined based on at least the model-specific indications by an aggregator. The aggregator may represent or define steps of a part of the method described herein. The step of determining the indication may be performed after (or "subsequently to") the steps of selecting the at least one model and determining the model-specific indications. As noted above, this may avoid determining model-specific indications which are not used for determining the (e.g., final) indication of the medical condition, thereby decreasing the amount of computing time and resources needed for determining the indication.

The (e.g., final) indication of the medical condition may be determined (e.g., by the aggregator) as an average of the model-specific indications, e.g., as a weighted average in which the weights are based on the at least one attribute, in particular the empirical performances. As noted above, this may enable prioritizing the models depending on the at least one attribute, and may thus improve reliability of the determined indication of the medical condition. The indication may be determined (e.g., by the aggregator) as a majority vote of discrete (e.g., binary) model-specific indications, for example. Depending on the use case, e.g., depending on the (e.g., type of) medical data at hand and/or depending on the medical condition to be determined, another vote of the model-specific indications may be used to determine the indication. For example, in case the medical condition to be determined is whether a patient is healthy or whether an anomaly is present in the medical data of the patient, it may be useful to determine that an anomaly is present if the model-specific indication of at least one of the selected models indicates so. This may increase the safety of use of the method. The indication may be determined (e.g., by the aggregator) based on a subset of the model-specific indications, wherein the subset may be determined based on the at least one attribute, in particular based on the result of the comparing. For example, a user may not trust predictions of a neural network and thus like to disregard model-specific indications determined by neural networks. In other words, model-specific indications of models having a low degree of explainability may be disregarded for determining the indication of the medical condition. Alternatively, such models may not be selected (e.g., by the selector) in the step of selecting the at least one model. As another example, several selected models may provide model-specific indications which all lie within a certain range, whilst individual models may determine erroneous outliers as the model-specific indications which lie outside the certain range. Model-specific indications which lie outside the certain range may be disregarded (e.g., by the aggregator) for determining the indication of the medical property. As mentioned before, this may allow a more reliable determination of the indication of the medical condition.

The (e.g. final) indication of the medical condition may, as noted above, be determined using an aggregation model. For example, the aggregator may define, comprise or consist of the aggregation model. The aggregation model may be generated by an aggregation model learning algorithm. The aggregation model learning algorithm may be a machine learning algorithm. As input for training the aggregation model, a plurality of model-specific indications may be used which are determined by the plurality of models based on a plurality of test instances. Optionally, together with the plurality of model-specific indications, at least one property associated with the aggregation training data may be used as input for training the aggregation model. The at least one property associated with the aggregation training data may be of the same type as the at least one property associated with the medical data and/or the at least one property associated with the training data. The aggregation model learning algorithm may be an algorithm that trains the aggregation model, such as a backpropagation algorithm if the aggregation model is a neural network, for example. The use of an aggregation model may improve reliability of the determined indication of the medical condition, especially when more information than only the plurality of model-specific indications (i.e., the at least one property) are considered.

Supervised learning algorithms may need labeled training data. A label may indicate whether a certain (e.g., the predetermined) medical condition or a set of medical conditions applies to the training data. A supervised learning algorithm may then be trained using the labeled training data in order to be able to predict, when applied to new data which has not been used for training, a label as the model-specific indication. At least one of the models comprised in the plurality of models may be generated by a supervised learning algorithm, e.g., using labeled training data (e.g., of at least one healthy patient and of at least one unhealthy patient with a disease).

Especially in medical diagnostic tasks, the structure of the labels to be predicted may be hierarchical. There may be higher-level labels (also referred to as class labels) for certain classes (e.g., "normal", corresponding to "healthy", or "anomalous", corresponding to "not healthy"). Some of the classes (e.g., the class "anomalous") may have several sub-classes (e.g., different subclasses for different diseases, such as "lung cancer", "breast cancer", "prostate cancer" and "melanoma", for example), which may be associated with lower-level labels (also referred to as subclass labels).

If a model is generated by a supervised learning algorithm using labeled training data, the model may only be able to provide (e.g., determine) a model-specific indication of whether a test instance is associated with one of the labels for which the model has been trained. In this case, a determination of a model-specific indication of the medical condition associated with the medical data of the test instance (e.g., the medical condition being "healthy" or "anomalous") may be possible only if the training data represents balanced amounts of all possible diseases that could potentially be found. In other words, labeled data of each possible sub-class may need to be available for training the supervised learning algorithm to enable a correct prediction of a label of the sub-class (e.g., "lung cancer") or the respective (e.g., higher-level) class (e.g., "anomalous"). Especially for diseases with very low prevalence, such training data may be hard to find or may be unavailable. Consequently, determining a model-specific indication of the medical condition associated with the medical data of the test instance (e.g., the medical condition being "healthy" or "anomalous") using a model generated by a supervised learning algorithm may be difficult in certain cases.

A reliable indication of the medical condition "healthy" or "anomalous", on the other hand, may allow a doctor to prioritize cases and allocate time appropriately even before having a look at the examination. Thus, in one particular variant, at least one of the models comprised in the plurality of models (e.g., the at least one selected model) may be generated, using (e.g., only) unlabeled training data (e.g., of healthy patients) by a learning algorithm, for example. In practical use cases, medical data taken from big register studies may be used as the unlabeled training data, for which it may be assumed that the majority of patients is generally healthy. The unlabeled training data may in this case also be referred to as "weakly labeled" training data due to the assumption that the majority of the plurality of patients is healthy. Instead of the unlabeled training data, training data having (e.g., only) the same (e.g., higher-level) label may be used to generate the at least one of the models comprised in the plurality of models (e.g., the at least one selected model). In the particular variant, the learning algorithm may be an unsupervised (e.g., machine) learning algorithm. The at least one of the models comprised in the plurality of models may be configured to provide (e.g., determine) an anomaly detection as the model-specific indication of the medical condition. In particular, at least one of the models comprised in the plurality of models may be generated by an unsupervised learning algorithm using (e.g., only unlabeled) training data of (e.g., only) healthy patients and, optionally, be configured to provide (e.g., determine) an anomaly detection as the model-specific indication of the medical condition. The anomaly detection may correspond to an out-of-distribution detection, for example. Using anomaly detection as an out-of-distribution detection for determining the model-specific indication may enable a reliable determination that a patient is unhealthy, even if training data representing the patient's disease has not been used for generating the at least one model. Still further, by using unlabeled training data of healthy patients, it may be easy to obtain a large set of training data.

The model-specific indication of the medical condition and/or the indication of the medical condition may comprise at least one result chosen from probabilities of an anomaly of different parts of a medical image comprised in the medical data, and a numerical value describing a probability of an anomaly of the overall medical data. Optionally, the numerical value may be derived from the probabilities of the anomaly for the different parts of the medical image. The model-specific indication and/or the indication may comprise a probability of a certain medical condition. This approach may be safe for use, as it may be capable of reflecting uncertainties in the determined model-specific indications and/or the determined indication.

In a first variant of the present disclosure, the determined model-specific indication and/or the determined (e.g., final) indication of the medical condition may comprise or be a probability of a predetermined medical condition being present in the test instance, for example. The probability may be a numerical value or a binary value. The (e.g., predetermined and/or models-specific) medical condition may be that a patient is healthy, that a patient is not healthy, that a patient has a certain (e.g., predetermined) symptom, that the patient exhibits a medical anomaly (e.g., compared to healthy patients), or the like.

In a second variant, the determined model-specific indication and/or the determined indication of the medical condition may comprise or be an identification of a part of the medical data that is associated with the (e.g., predetermined) medical condition. For example, the model-specific indication of the medical condition and/or the indication of the medical condition may comprise or be an identification of a part of the medical image comprised in the medical data, which part may be associated with the (e.g., predetermined) medical condition (e.g., a part of the image which is associated with cancer tissue). A part of an image (e.g., the medical image) may be a pixel, a voxel, an area or a volume, for example.

In a variant combining the first and the second variant, the determined model-specific indication and/or the determined indication of the medical condition may comprise or be a probability of a part of the medical data (e.g., a part of the medical image) being associated with the (e.g., predetermined) medical condition.

The method may further comprise determining (e.g., by the selector and/or by the aggregator) that a reliable determination of the indication is impossible, if the at least one property associated with the medical data of the test instance does not indicate suitability of the at least one model (e.g., suitability for determining a model-specific indication for the medical data of the test instance). The step of determining that a reliable determination of the indication is impossible may be based on a result of the comparing. For example, if the result of comparing is a degree of suitability, it may be determined that a reliable determination of the indication is impossible if the degree of suitability fulfils a predetermined criterion (e.g., lies below a predetermined threshold). In one variant, in case no suitable model can be selected, it may be determined that a reliable determination of the indication is impossible. In case it is determined that a reliable determination of the indication is impossible, the method may not determine the model-specific indication and/or the indications, and/or the method may comprise triggering output of a notification on an output device, the notification informing a user that a reliable determination of the indication is impossible. In this way, a determination of an unreliable indication of the medical condition may be prevented.

In case it is determined that a reliable determination of the indication of the medical condition is possible, or in case the indication of the medical condition has been determined, the method may comprise triggering output of a notification on an output device, the notification informing a user of the indication or that a reliable determination of the indication is possible. The notification and/or the indication may be visualized on a display accordingly.

The method may further comprise a step of determining (or "making"), based on at least the determined indication, a medical diagnosis. The determination of the medical diagnosis may be further based on values described by at least a part of the medical data which part is referred to or described by the indication. The determination of the medical diagnosis may comprise comparing the determined indication with a set of medical diagnoses correlated with different indications and, for example, choosing a (e.g., matching or most suitable) medical diagnosis from the set of diagnoses based on the comparing. The medical diagnosis may be, for example, that a patient has a brain tumor, a region or volume of cancerous tissue in the patient's body, that a patient is healthy, that a patient has amyotrophic lateral sclerosis (ALS), or the like.

In one implementation, at least one of the models comprised in the plurality of models, in the following also referred to as density model, may correlate parts of the medical image comprised in or represented by the medical data with parts of a reference image. For example, a (e.g., predetermined) first registration between the medical image and the reference image may be applied to relate both the medical image and the reference image to a common frame of reference. The first registration may comprise a transformation transforming a coordinate system of the (e.g., part of the) medical image to a coordinate system of the (e.g., part of the) reference image, or vice versa. The first registration may comprise or consist of a first (image) transformation. The first registration may be applied to one or more parts of the medical image and the reference image only. Different first registrations may be applied to different parts of the medical image and the reference image. In one variant, the medical image may be matched to the reference image to correlate the parts of the medical image with the parts of the reference image. The matching may be performed based on an image fusion algorithm, for example. The reference image may be a medical image, an image of a generic anatomical model, an image obtained by averaging a plurality of patient images registered to a common frame of reference, or the like.

The density model may further compare (e.g., provide a comparison of) an image value (e.g., at least one image value or all image values) of at least one part of the medical image comprised in or represented by the medical data with information associated with a correlated part of the reference image to obtain the model-specific indication of the medical condition.

The information with which the image value is compared may be or may have been generated or determined by a learning algorithm defining a sequence of method steps. For example, the information with which the image value is compared may be or may have been generated or determined (e.g., on a different processor than the processor or apparatus performing the method of the first aspect and/or before performing the method of the first aspect) by matching a plurality of training images to a base image to correlate parts of each of the training images with parts of the base image. The matching may be performed using an image fusion or image matching algorithm, for example. Alternatively, or additionally, the matching may comprise or consist of applying a (e.g., predetermined) second registration between (e.g., a part of) each of the plurality of training images and (e.g., a part of) the base image to relate (e.g., the part of) each of the plurality of training images and (e.g., the part of) the base image to a common frame of reference. The second registration may comprise a transformation transforming a coordinate system of one of the (e.g., part of the) training image to a coordinate system of the (e.g., part of the) base image or vice versa. The second registration may thus comprise or consist of a second (image) transformation. The second registration may be applied to one or more parts of each of the training images and one or more parts of the reference image only. Different second registrations may be applied to different parts of each of the training images and the reference image.

The information with which the image value is compared may be or may have been generated or determined (e.g., on the different processor and/or before performing the method of the first aspect) further by determining image values of at least one part of each of the plurality of training images correlated with a part of the base image, wherein the part of the base image may be assigned to (e.g., correlated to, mapped to or matched to) the correlated part of the reference image using a (e.g., third) predetermined transformation. The part of the base image may be assigned to the correlated part of the reference image using a predetermined third registration. The third registration may comprise a transformation transforming a coordinate system of the (e.g., part of the) base image to a coordinate system of the (e.g., part of the) reference image, or vice versa. The third registration may thus comprise or consist of a third (image) transformation. The predetermined third transformation or registration may relate both (e.g., the correlated part of) the reference image and (e.g., the part of) the base image to a common frame of reference.

The image values of the at least one part may be values of a single pixel or voxel of each of the respective training images (e.g., density values, Hounsfield values, color values, brightness values, saturation values, etc.). The image values of the at least one part may be an average, maximum or minimum of pixel or voxel values of pixels or voxels comprised in the at least one part of each of the respective training images. The information may be a collection of (e.g., average, maximum or minimum) pixel or voxel values of corresponding parts of the plurality of training images which are correlated with the part of the base image.

The information with which the image value is compared may be or may have been generated or determined (e.g., on the different processor and/or before performing the method of the first aspect) further by determining the information based on the determined image values of the at least one part of each of the plurality of training images. The information may comprise, be or consist of a statistical distribution function of image values of the at least one part of the plurality of training images, for example. The statistical distribution function may be determined as a best fit of a predetermined distribution type (e.g., Gaussian distribution, kernel density estimation, polynomic distribution, etc.) to the image values of the at least one part of the plurality of training images (e.g., the collection of pixel or voxel values). Experiments have shown that the density model, using information comprising the statistical distribution function, may result in a highly reliable determination of the model-specific indication.

Alternatively, or additionally, the information may comprise, be or consist of an average image value of the at least one part of all of the plurality of training images, e.g., an average of the collection of pixel or voxel values. The information may (e.g., further) comprise, be or consist of a mean deviation of the image values of the at least one part of all of the plurality of training images from the average image value, e.g., a mean deviation of each of the collection of pixel or voxel values from the average of the collection of pixel or voxel values. Experiments have shown that the density model, using information comprising the average image value and the mean deviation, may result in a reliable determination of the model-specific indication. Also, such a density model may be generated in a fast manner with a low amount of computing resources.

According to a second aspect, a medical data processing for determining an indication of a medical condition is provided. The method comprises correlating parts of a medical image comprised in medical data of a test instance with parts of a reference image. The method further comprises comparing an image value of at least one part of the medical image with information associated with a correlated part of the reference image to obtain the indication of the medical condition. For example, the information is or has been generated or determined (e.g., on a different processor than the processor performing the method of the second aspect and/or at a different time) by matching a plurality of training images to a base image to correlate parts of each of the training images with parts of the base image, determining image values of at least one part of each of the plurality of training images correlated with a part of the base image, wherein the part of the base image is assigned to the correlated part of the reference image using a predetermined transformation, and determining the information based on the determined image values of the at least one part of each of the plurality of training images, wherein the information is a statistical distribution function of image values of the at least one part of the plurality of training images.

All details and variants of the features of the first aspect denoted with the same terminology as used for the second aspect may also apply to the method of the second aspect, wherein the indication of the second aspect may correspond to the model-specific indication of the first aspect. The method of the second aspect may also comprise the step of determining, based on the determined indication, the medical diagnosis. The method of first aspect may be combined with the method of second aspect, and vice versa. The method of the second aspect may be used to determine the respective model-specific indication of the first aspect. That is, the method of the second aspect may correspond to a method performed by one of the plurality of models of the method of the first aspect.

The method of the first aspect and/or the method of the second aspect may comprise a step of acquiring the medical data. The step of acquiring the medical data may only consist of acquiring data from a storage medium. The step of acquiring medical data may not be practiced on a human or animal body. In particular, none of the steps comprised in the method of the first aspect and/or the method of the second aspect may require an interaction with the human or animal body. None of the steps comprised in the method of the first aspect and/or the method of the second aspect may necessitate the presence of the body of the patient.

According to a third aspect, an apparatus is provided. The apparatus comprises at least one processor and at least one memory, the at least one memory containing instructions executable by the at least one processor such that the apparatus is operable to perform the method of the first aspect and/or the method of the second aspect. The apparatus may further comprise an output device and/or an interface for receiving, obtaining, transmitting or sending data or information. The method of the first aspect and/or the method of the second aspect may be a computer-implemented method. Each of the steps of the method of the first aspect and/or the method of the second aspect may be performed by the at least one processor of the apparatus.

According to a fourth aspect, a computer program product is provided. The computer program product comprises program code portions for performing the method of the first aspect and/or the second aspect when the computer program product is executed on one or more processors. The computer program product may be stored on one or more (e.g., non-transitory) computer readable recording media. The computer program product may be contained in a carrier such as an electronic signal, an optical signal, a radio signal or a data stream.

According to a fifth aspect, one or more computer readable recording media are provided, the one or more computer readably recording media storing the computer program product of the fourth aspect. The one or more computer readable recoding media may be non-transitory storage media.

### Brief Description of the Drawings

Further details and advantages of the technique presented herein will be described with reference to exemplary implementations illustrated in the figures, in which:
- Figs. 1: illustrates an exemplary composition of an apparatus according to the present disclosure;
- Fig. 2: illustrates a method of determining an indication of a medical condition, which may be performed by the apparatus according to the present disclosure;
- Fig. 3: schematically illustrates an exemplary method of generating a model, which may be performed by the apparatus according to the present disclosure;
- Fig. 4: schematically illustrates an exemplary method of determining a model-specific indication of a medical condition, which may be performed by the apparatus according to the present disclosure;
- Fig. 5: schematically illustrates an exemplary method of generating a plurality of models, which may be performed by the apparatus according to the present disclosure;
- Fig. 6: schematically illustrates an exemplary method of determining an indication of a medical condition, which may be performed by the apparatus according to the present disclosure;
- Fig. 7: schematically illustrates exemplary labels of data;
- Fig. 8: schematically illustrates an exemplary prevalence of different diseases; and
- Fig. 9: illustrates a method which may be performed by the apparatus according to the present disclosure.

### Detailed Description

In the following description, for purposes of explanation and not limitation, specific details are set forth in order to provide a thorough understanding of the present disclosure. It will be apparent to one skilled in the art that the present disclosure may be practiced in other implementations that depart from these specific details.

Those skilled in the art will further appreciate that the steps, services and functions explained herein below may be implemented using individual hardware circuitry, using software functioning in conjunction with a programmed micro-processor or general-purpose computer, using one or more Application Specific Integrated Circuits (ASICs) and/or using one or more Digital Signal Processors (DSPs). It will also be appreciated that when the present disclosure is described in terms of a method, it may also be embodied in one or more processors and one or more memories coupled to the one or more processors, wherein the one or more memories are encoded with one or more programs that perform the steps, services and functions disclosed herein when executed by the one or more processors.

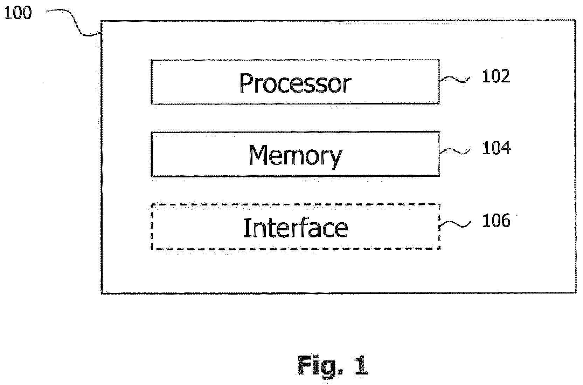

Fig. 1 illustrates an exemplary composition of an apparatus 100 according to the present disclosure. The apparatus 100 comprises a processor 102, a memory 104 and, optionally, an interface 106. The processor 102 is coupled to the memory 104 and, optionally, to the interface 106. The interface 106 is configured to obtain, receive, send or transmit data from or to an external unit such as a data storage unit, a server, a user input unit, an output unit such as a display or a speaker, or the like. The interface 106 may be configured to send a trigger signal to the output unit to trigger output of an acoustic and/or optical notification (message) for a user. The memory 104 is configured to store a program comprising instructions which, when executed by the processor 102, cause the processor 102 to carry out a method as described herein. The program may be stored on a storage medium.

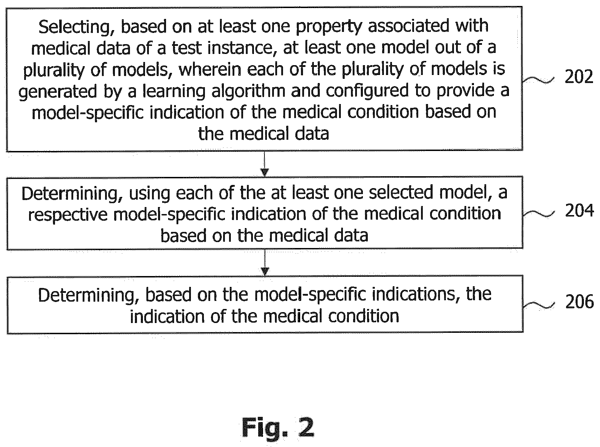

Fig. 2 illustrates a method which may be performed by the apparatus 100. The method of Fig. 2 may correspond to the method of the first aspect described above. The method comprises a step 202 of selecting (e.g., by or using the selector described herein), based on at least one property associated with medical data of a test instance, at least one model out of a plurality of models, wherein each of the plurality of models is generated by a learning algorithm and configured to provide (e.g., determine) a model-specific indication of the medical condition based on the medical data. Each of the models may be referred to as a base learner model.

The method further comprises a step 204 of determining, using (e.g., only) each of the at least one selected model, a respective model-specific indication of the medical condition based on the medical data.

The method also comprises a step 206 of determining (e.g., by or using the aggregator described herein), based on the model-specific indications, the (e.g., final) indication of the medical condition.

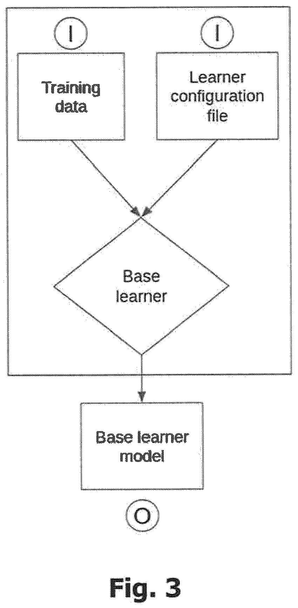

Fig. 3 schematically illustrates an exemplary method of generating a base learner model according to the present disclosure. The method shown in Fig. 3 may be performed by the apparatus 100 or by a different apparatus (not shown) comprising a processor and a memory storing instructions which, when executed by the processor, cause the processor to perform the method shown in Fig. 3. The method shown in Fig. 3 may be used for generating at least one of the plurality of models used in the method of Fig. 2.

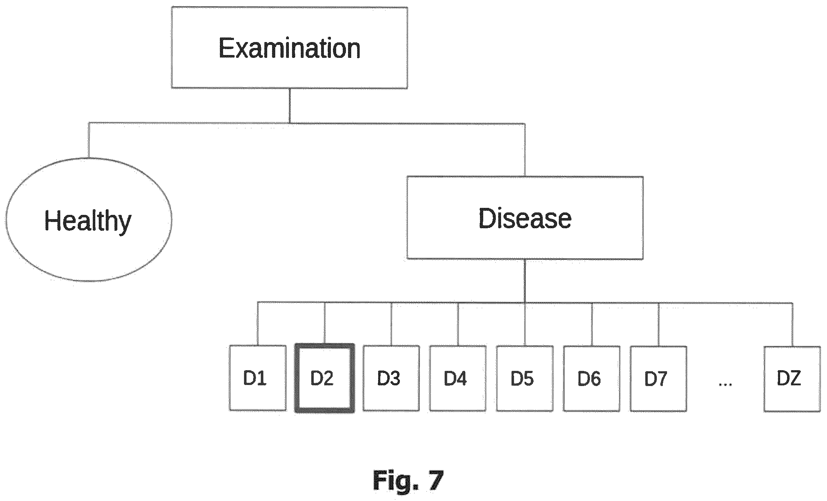

Training data, such as medical images of a plurality of patients, for example, may be used by a learning algorithm, also referred to herein below as base learner, to generate one of the base learner models. A learner configuration ("config") file may define the type and/or structure of the base learner model. The learner configuration file may comprise at least one hyperparameter defined in or used by the (e.g., unlearned) base learner model. The learner configuration file may define the learning algorithm used to generate the base learner model and, optionally, hyperparameters defined in or used by the learning algorithm. Some preferred examples of base learners and base learner models will be described in detail with reference to Figs. 7-9 below. In the method shown in Fig. 3, the base learner model may be generated based on the training data and the learner configuration file. For that reason, the training data may be used as an input "I", the learner configuration file may be used as an input "I" and the base learner model may be the determined output "O". The base learner model may be regarded as the output of the base learner.

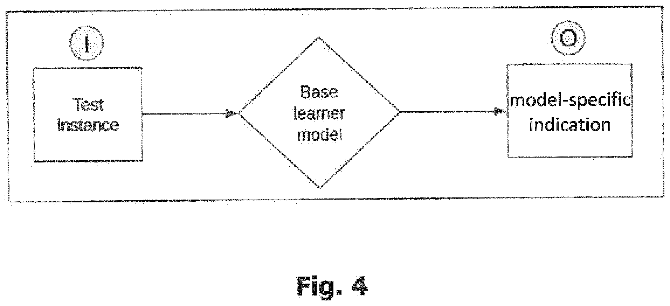

Fig. 4 schematically illustrates an exemplary method of determining the model-specific indication of the medical condition according to the present disclosure. The method shown in Fig. 4 may be performed by the apparatus 100 and be part of the method shown in Fig. 2, in particular part of step 204. In the method shown in Fig. 4, the model-specific indication of the medical condition may be determined based on a test instance, for example, based on medical data of the test instance. For that reason, the test instance, in particular the medical data of the test instance, may be used as the input "I" and the model-specific indication may be determined as the output "0" using the base learner model.

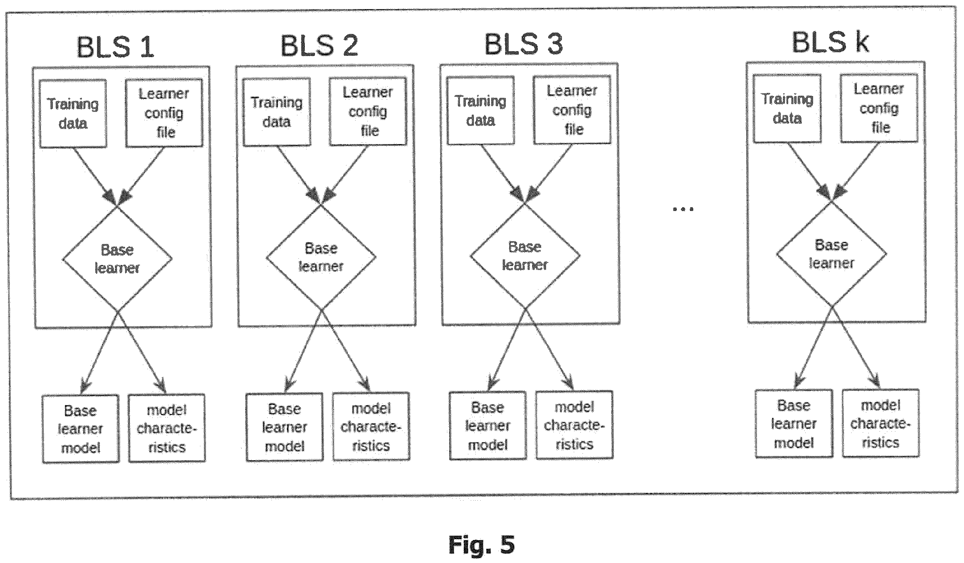

Fig. 5 schematically illustrates an exemplary method of generating a plurality of models according to the present disclosure. The method may be performed by the apparatus 100 or by the different apparatus. The method of Fig. 5 may be based on the method of Fig. 3. In particular, also in this case, training data and a learner configuration file may be used to determine a base learner model. As shown in Fig. 5, a plurality of different base learner models BLS 1 to BLS k may be determined. The base learner models BLS1 to BLS k may differ from one another by at least one of the training data and the learner configuration file being used to generate the respective base learner model. Using a predefined set of (e.g., labeled) medical data, each of the plurality of base learner models BLS1 to BLS k may be tested to obtain respective model characteristics. The respective model characteristics may be empirical performances of a base learner model (e.g., temporal performance, probability of false negative or false positive detections etc.), for example. The model characteristics may include a property associated with the learning data used for generating the respective base learner model. The model characteristics may include a degree of explainability of the respective base learner model. The plurality of base learner models BLS 1 to BLS k shown in Fig. 5 may be used in the method of Fig. 2 as the plurality of models.

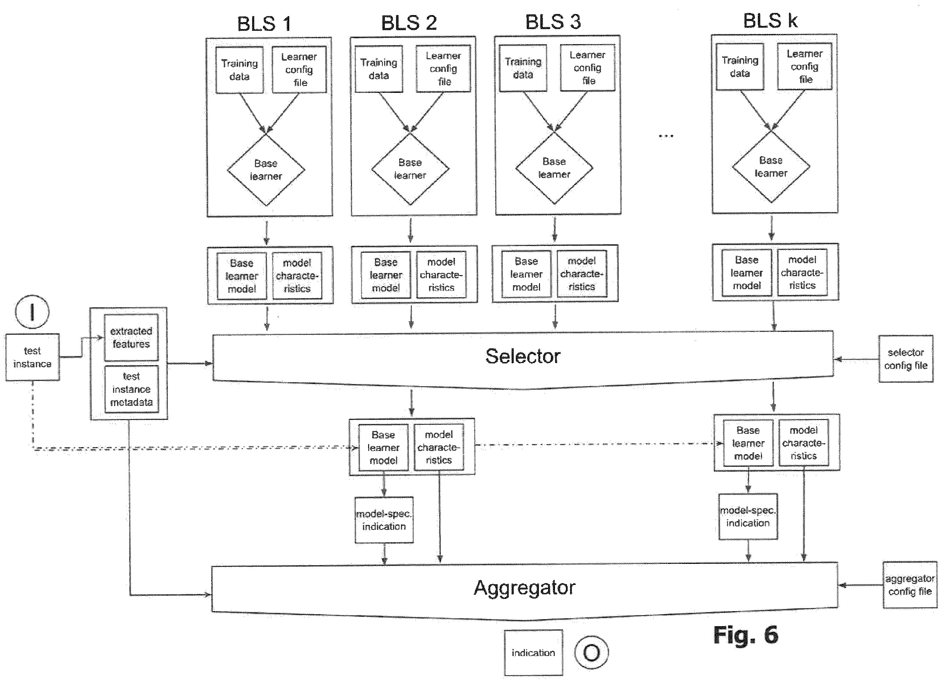

Fig. 6 schematically illustrates an exemplary method of determining an indication of a medical condition according to the present disclosure. This method may correspond to the method of Fig. 2. The method may be performed by the apparatus 100. The plurality of base learner models BLS 1 to BLS k may be determined according to the method of Fig. 5.

A test instance, in particular medical data of a test instance, may be used as input for a selector which may select the at least one model from the plurality of base learner models BLS 1 to BLS k. The selector may correspond to the selector described above with reference to the method of the first aspect. The selector may be defined by a selector configuration ("config") file. The selected models may then be used to determine the respective model-specific indications. In the shown example, the base learner model BLS 2 and the base learner model BLS k may be selected from the plurality of base learner models BLS 1 to BLS k. In this example, only the selected based learner models BLS 2 and BLS k may be used to determine the respective model-specific indications, as indicated in Fig. 6. The selection of the at least one model may be performed before the model-specific indications are determined and before the indication is determined.

Based on the model-specific indications, the (e.g., final) indication of the medical condition may be determined, for example by or using an aggregator. The aggregator may correspond to the aggregator described above with reference to the method of the first aspect. The aggregator may determine or provide the (e.g., final) indication based on the determined model-specific indications, the model characteristics, test instance metadata and features extracted from the test instance. The aggregator may be defined by an aggregator configuration ("config") file. Note that the aggregator may not necessarily be a hardware component but may be embodied in software. The aggregator configuration file may define how the inputs to the aggregator are combined into the output "O", namely, into the indication of the medical condition. The aggregator may comprise or consist of the aggregation model described herein above. The aggregation model may be defined by the aggregator configuration file. Not all of the inputs shown in Fig. 6 may be used together with the model-specific indications to determine the indication of the medical condition. For example, the indication may be determined only based on the model-specific indications and the test instance metadata.

As mentioned with reference to Fig. 2, preferably, at least one of the base learner models BLS 1 to BLS k may be selected based on the at least one property associated with the medical data of the test instance. The at least one property associated with the medical data may comprise a feature of a medical image comprised in the medical data, which corresponds to the "extracted features" shown in Fig. 6 and may be a slice thickness of the medical image, a contrast of the medical image, an imaging modality (e.g., CT or MR) of the medical image or a radiomics feature of the medical image, for example. The radiomics feature may be extracted from the medical image using the software package "pyradiomics" available on https://github.com/Radiomics/pyradiomics, for example. The at least one property associated with the medical data may comprise a characteristic of a patient to which the medical data (and, e.g., the test instance) relates, which corresponds to the "test instance metadata" shown in Fig. 6. The test instance metadata may be comprised in a header of the medical image of the test instance. The characteristic may be an age, an ethnicity or a gender of the patient to which the medical data relates, for example.

The step of selecting may comprise comparing, individually for each of the plurality of models, the at least one property associated with the medical data of the test instance and at least one property associated with the training data used for generating the individual model. The comparing may result in a degree of suitability for each of the plurality of models. For example, models that have been trained with training images having a low slice thickness may have a low degree of suitability when compared with a higher slice thickness of the medical image of the test instance. Models that have been trained with training images having a certain contrast may have a low degree of suitability when compared with a contrast of the medical image of the test instance which deviates from the certain contrast more than a predefined threshold. Also, suitability of a base learner model that has been trained with training data of patients above the age of 50 may be low for medical data of the test instance which relates to a 5-year-old patient. As a further example, suitability of a base learner model that has been trained only with training data of Asian patients may be low for medical data of the test instance which relates to a patient of another ethnicity such as an African or a European patient. Also, suitability of a base learner model that has been trained only with training data of female patients may be low for medical data of the test instance which relates to a male patient.

The method may further comprise determining that a reliable determination of the indication is impossible, if the at least one property associated with the medical data of the test instance does not indicate suitability of the at least one model. In this case, a notification such as a warning tone or a visual warning may be triggered to be output on the output device, e.g., by sending a corresponding trigger signal via the interface 106 to the output device.

The indication may be determined (e.g., by or using the aggregator) based on a majority vote of the model-specific indications or based on a mean aggregation for continuous numerical model-specific indications. Other aggregation functions are possible. For example, in case the indication shall be very sensitive, the indication may be that the patient to which the medical data of the test instance relates is unhealthy, if one or more of the model-specific indications indicate such unhealthiness.

The indication may be determined further based on at least one of attribute chosen from a result of the comparing, empirical performances (e.g., comprised in the model characteristics) of each of the plurality of models and a degree of explainability (e.g., comprised in the model characteristics) of each of the plurality of models. The degree of explainability may be determined based on the learner configuration file or predetermined and indicates how well a user is able to understand the functioning of the base learner (model). The degree of explainability may be obtained from a database.

For example, only model-specific indications may be taken into account (e.g., used or considered) for determining the (e.g., final) indication, attributes of which fulfil a predetermined criterion. In case no attribute fulfils the predetermined criterion, it may be determined that a reliable determination of the indication is impossible and a corresponding notification may be output as noted above.

Alternatively, a weighted average of the model-specific indications may be determined as the (e.g., final) indication of the medical condition. The weights of the respective model-specific indications may be determined based on the attribute, in particular based on the empirical performances. Weights w_i (e.g., that sum up to 1) may be defined for each of the learner models BLS1 to BLS k and proportional to the base learner's empirical performance. For example, w_i may be proportional to exp(p_i), wherein p_i may be the empirical performance of base learner model i.

Still alternatively, the (e.g., final) indication may be determined using an aggregation model, e.g., the aggregation model described herein above. In particular, the aggregator may comprise or consist of the aggregation model described herein above and/or be generated by a supervised learning algorithm, for example. The supervised learning algorithm may be trained with a set of determined model-specific indications, and optionally, a set of model characteristics, test instance metadata and/or extracted features and the generated aggregation model may be capable of providing the indication of the medical condition based on the inputs of the "aggregator". The aggregation model learning algorithm may be defined by the aggregator configuration file, for example.

The indication of the medical condition may be used to trigger output of a notification on the output device, the notification informing the user of the indication. For example, the interface 106 may output a trigger signal to a display which then displays a visualization of areas of the medical image comprised in the medical data of the test instance, which areas exhibit the medical condition. Alternatively, a score or a binary visualization such as a color red or green may be displayed informing the user of the indication of the medical condition. In one example, the indication itself may be visualized on the display. Alternatively, or additionally, a medical diagnosis may be determined based on the indication of the medical condition. For example, if the indication of the medical condition represents an anomalous volume in a medical image, further properties of the anomalous volume such as image values, color values, volume size or else may be taken into account (e.g., used or considered) for determining the diagnosis, e.g., the diagnosis that the patient to which the medical data relates has a brain tumor.

As will be apparent for the skilled person, other ways of determining the (e.g., final) indication based on the model-specific indications may be possible. In the following, examples of base learners and base learner models will be described.

Generally, supervised learning algorithms may need labeled training data. A label may indicate whether a certain medical condition or a set of medical conditions applies to the training data. A supervised learning algorithm may then be trained using the labeled training data in order to be able to predict, when applied to new data which has not been used for training, a label as the model-specific indication. At least one of the base learner models BLS 1 to BLS k may in one variant be generated by a supervised learning algorithm using labeled training data (e.g., of at least one healthy patient and of at least one unhealthy patient with a disease).

Especially in medical diagnostic tasks, the structure of the labels to be predicted may be hierarchical. As shown in Fig. 7, there may be higher-level labels (also referred to as class labels) for certain classes (e.g., "normal", which corresponds to "Healthy" or "anomalous", which corresponds to "Disease" or "not healthy"). Some of the classes (e.g., the class "Disease") may have several sub-classes (e.g. different subclasses D1 to DZ for different diseases, for example "lung cancer", "breast cancer", "prostate cancer" and "melanoma") which may be associated with corresponding lower-level labels (also referred to as subclass labels).

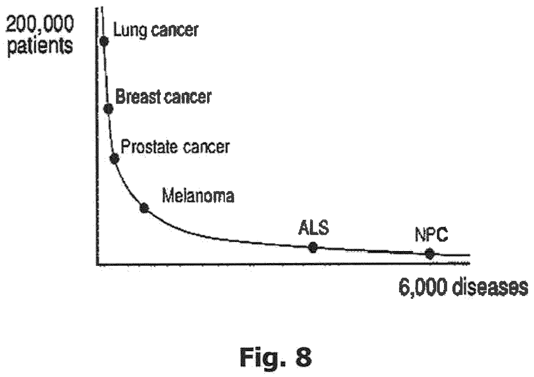

If a model is generated by a supervised learning algorithm using labeled training data, the model may only be able to provide (e.g., determine) a model-specific indication of whether a test instance is associated with one of the labels for which the model has been trained. In this case, a determination of a model-specific indication of the medical condition associated with the medical data of the test instance, the medical condition being "healthy" or "disease", may be possible only if the training data represents balanced amounts of all possible diseases D1 to DZ that could potentially be found. In other words, labeled data of each possible subclass D1 to DZ may need to be available for training the supervised learning algorithm to enable a correct prediction of a lower-level label of the subclass (highlighted subclass "D2" in Fig. 7 which may correspond to "lung cancer") or the respective higher-level class ("disease" in Fig. 7). Especially for diseases with very low prevalence (see amyotrophic lateral sclerosis, ALS, and nasopharyngeal carcinoma, NPC, in Fig. 8), such training data may be hard to find or unavailable. Consequently, determining a model-specific indication of the medical condition using a model generated by a supervised learning algorithm, may be difficult in certain cases.

A reliable machine-based indication of the medical condition "healthy" or "anomalous" (or "disease") may allow a doctor to prioritize cases and allocate time appropriately even before having a look at the examination. As noted above, the method described herein may further comprise determining a medical diagnosis based on the indication of the medical condition, thereby improving a medical workflow for the doctor.

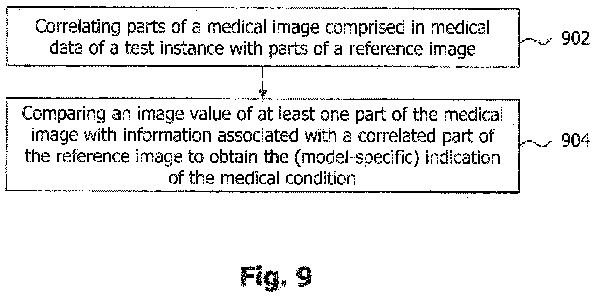

In view of the above, a method of determining the model-specific indication of the medical condition may be provided, as schematically illustrated in Fig. 9. Note that the model-specific indication may be directly used as the indication of the medical condition and thus, the method may be a method of determining an indication of a medical condition. The method may be performed by the apparatus 100.

The method comprises a step 902 of correlating parts of a (e.g., the) medical image comprised in (e.g., the) medical data of a (e.g., the) test instance with parts of a reference image, for example, using the first registration described above.

The method also comprises a step 904 of comparing an image value of at least one part of the medical image with information associated with a correlated part of the reference image to obtain the indication of the medical condition.

The information has been generated, e.g., by the different apparatus, by: matching a plurality of training images to a base image (e.g., using the second registration described above) to correlate parts of each of the training images with parts of the base image, determining image values of at least one part of each of the plurality of training images correlated with a part of the base image, wherein the part of the base image is assigned to the correlated part of the reference image using a predetermined transformation (e.g., the third registration described above), and determining the information based on the determined image values of the at least one part of each of the plurality of training images.

The base image may be an atlas image generated based on a plurality of medical images. The base image may be determined by applying a rigid registration of a plurality of medical images to a common frame of reference and averaging image values of all these medical images. By applying the rigid registration, each part of each of the patient images may be correlated with the common frame of reference. That is, the rigid registration may comprise a transformation matrix describing a transformation of a coordinate system of a patient image into a common coordinate system. Different rigid registrations may be used for different ones of the plurality of medical images. The base image may be an MR atlas image. CT images of the training data may be matched to the MR atlas image using mattes-mutual-information. In one variant, each of the learning images may be first rigidly and affinely transformed to the base image before the SyN non-linear transformation may be applied. Each of the aforementioned registration steps may be iteratively repeated until a convergence or a maximum number of iteration is reached. Also, each of these steps may be performed on different levels of resolution, starting with a low resolution and then going to higher resolutions, to obtain a coarse registration which is then improved to a fine-grained registration.

A dataset of a plurality of 3D CT images (dimensions m x n x p) of healthy patients may be mapped as the training images to the base image which may be a CT volume. This mapping may be performed by applying a registration (e.g., the second registration described above) between each of the plurality of 3D CT images of healthy patients to the base image. By applying the registration, each part of each of the patient images may be correlated with parts of the base image. That is, the registration may comprise a transformation matrix describing a transformation of a coordinate system of a patient image into a coordinate system of the base image and/or a plurality of transformations of different parts of the patient image into the coordinate system of the base image. Of course, different registrations may be used for different patient images. The registration may be determined using a symmetric diffeomorphic image registration with cross-correlation and a SyN algorithm comprised in the Advanced Normalisation Tools library available on https://github.com/ANTsX/ANTs, for example.

In a first implementation, the information may comprise or be a statistical distribution function of image values of the at least one part of the plurality of training images. The information may have been determined as follows: For each voxel position x_{a,b,c} with 1<=a<=m, 1<=b<=n, 1<=c<=p, a statistical distribution function may be fitted over all voxel values of all training images. The distribution may be a Gaussian distribution or a kernel density distribution (KDE), for example. This may result in m x n x p distribution functions p_{a,b,c} that have been independently estimated based on the learning images.

The base learner model of the first implementation may use the medical data to determine or provide the model-specific indication of the medical condition as follows: When the model-specific indication of the medical condition is to be determined based on the medical image, the medical image may first be registered to the reference image which is registered to the base image with a predetermined transformation or which is the base image. If a voxel value of the medical image is higher than the upper q/2 quantile or lower than the lower q/2 quantile as defined by the corresponding distribution function, this voxel may be tagged or identified as anomalous. A percentile filter may be used to smooth the results, and thresholding may be used to obtain a binary segmentation mask. The binary segmentation mask may indicate parts of the medical image of the test instance which are anomalous. The binary segmentation mask may be determined as the model-specific indication. An overall anomaly score for the medical image of the test instance may be determined, e.g., as the model-specific indication, by counting the number of anomalous voxels in the medical image or by dividing this number by the total number of voxels of the medical image.

In a second implementation, the information may comprise an average image value of the at least one part of all of the plurality of training images and, optionally, a mean deviation of the image values of the at least one part of all of the plurality of training images from the average image value. The information may have been determined as follows: For each voxel position x_{a,b,c} with 1<=a<=m, 1<=b<=n, 1<=c<=p, a voxel value may be obtained of all training images. For each voxel position, an average voxel value of all training images may be determined. In other words, the training images may be averaged voxel-wise. Beforehand, the training images may be normalized so that all voxel values lie in a predetermined range (e.g., [0, 1]). In addition, a mean error map may be computed by calculating an average over the voxel-wise difference of all training images to the average voxel values.

The base learner model of the second implementation may use the medical data to determine or provide the model-specific indication of the medical condition as follows: When the model-specific indication of the medical condition is to be determined based on the medical image, the medical image is first registered to the reference image which is registered to the base image with a predetermined transformation or which is the base image. The absolute difference of the medical image to the average voxel values may then be determined per voxel. The mean error map may be subtracted from the absolute differences. A percentile filter may be used to smooth the results, and thresholding may be used to obtain a binary segmentation mask. The binary segmentation mask may indicate parts of the medical image of the test instance which are anomalous. The binary segmentation mask may be determined as the model-specific indication. An overall anomaly score for the medical image of the test instance may be determined, e.g., as the model-specific indication, by counting the number of anomalous voxels in the medical image or by dividing this number by the total number of voxels of the medical image.

The method of Fig. 9 may be combined with the method described above with reference to Figs. 2 to 6. In particular, at least one of the base learner models BLS 1 to BLS k described above may provide (e.g., be capable of) the functionality of the method of Fig. 9, e.g., correspond to the density model described above. The indication of the medical condition determined by the method of Fig. 9 may be used as the model-specific indication in the method described above with reference to Figs. 2 to 6. In other words, at least one of the models comprised in the plurality of base learner models BLS 1 to BLS k may be generated by an unsupervised learning algorithm using unlabeled training data of healthy patients and configured to provide (e.g., determine) an anomaly detection as the model-specific indication of the medical condition.

As explained above, the model-specific indication of the medical condition and/or the (e.g., final) indication of the medical condition may comprise at least one result chosen from probabilities of an anomaly for different parts of a medical image comprised in the medical data and a numerical value describing a probability of an anomaly of the overall medical data, wherein the numerical value is optionally derived from the probabilities of the anomaly for the different parts of the medical image.

As has become apparent from the above, the present disclosure provides a technique for determining an indication of a medical condition. At least one of the base learner models may be generated using unlabeled training data of healthy patients. Such a base learner model may provide (e.g., determine) a model-specific indication of a medical condition, which may be a score indicating whether a new, prior unseen test instance is in- or out-of-distribution compared to the distribution of the training data. Accordingly, (e.g., only) training data of healthy patients, which is easily obtainable, may be used to generate the at least one model.

A plurality of the base learner models may represent an ensemble. A (most suitable) subset of a plurality of base learner models may be selected based on the test instance at hand. In other words, models to be included in the ensemble may be dynamically selected based on the medical data. This may enable a more reliable determination of the model-specific indications. Model-specific indications of the selected base learner models may be aggregated to determine the indication of the medical condition. The aggregation may further improve reliability of the prediction (i.e., of the determined indication).

Especially if the base learners' errors are uncorrelated, combining them in an ensemble may enhance robustness. Further, model-specific indications may be combined in such a way that explainable base learners are preferred over non-explainable deep learning models. This may result in higher interpretability of the model-specific indications. False negative predictions may be avoided by the selection of the at least one base learner model and the determination of the indication based on the model-specific indications.

By using learning data of a higher-level "normal" class (that has no or only a few subclasses, e.g., learning data of only healthy patients), a robust, reliable indication of whether the medical data of the test instance belongs to an in-distribution-instance ("normal" or "healthy" instance) or not may be possible. Through the coupling mechanism (rule-based or learned) by the proposed ensemble, in particular, by determining the indication based on the model-specific indications, an advanced predictive performance, as well as increased robustness of the predictions may be ensured. The techniques described herein may be applicable to any type of diagnostic procedure, and may provide a prediction with respect to the higher-level classes without having to use training data with a detailed sub-class labeling, and even without having to use training data of out-of-distribution samples.

It is believed that the advantages of the technique presented herein will be fully understood from the foregoing description, and it will be apparent that various changes may be made in the form, constructions and arrangement of the exemplary aspects thereof without departing from the scope of the disclosure or without sacrificing all of its advantageous effects. Because the technique presented herein can be varied in many ways, it will be recognized that the disclosure should be limited only by the scope of the claims that follow.

## Claims

1. A medical data processing method for determining an indication of a medical condition, the method comprising:
selecting (202), based on at least one property associated with medical data of a test instance, at least one model out of a plurality of models (BLS 1 - BLS k), wherein each of the plurality of models (BLS 1 - BLS k) is generated by a learning algorithm and configured to provide a model-specific indication of the medical condition based on the medical data;
determining (204), using each of the at least one selected model, a respective model-specific indication' of the medical condition based on the medical data; and
determining (206), based on the model-specific indications, the indication of the medical condition.

2. The method of claim 1, wherein the at least one property associated with the medical data comprises a feature of a medical image comprised in the medical data.

3. The method of claim 1 or 2, wherein the at least one property associated with the medical data comprises a characteristic of a patient to which the medical data relates.

4. The method of any of claims 1 to 3, wherein the step of selecting comprises comparing, individually for each of the plurality of models (BLS 1 - BLS k), the at least one property associated with the medical data of the test instance and at least one property associated with training data used for generating the individual model.

5. The method of claim 4, wherein the indication is determined further based on at least one attribute chosen from a result of the comparing, empirical performances of each of the plurality of models (BLS 1 - BLS k) and a degree of explainability of each of the plurality of models.

6. The method of any of claims 1 to 5, wherein at least one of the models comprised in the plurality of models (BLS 1 - BLS k) is generated by an unsupervised learning algorithm using unlabeled training data of healthy patients and, optionally, configured to provide an anomaly detection as the model-specific indication of the medical condition.

7. The method of any of claims 1 to 6, wherein the model-specific indication of the medical condition and/or the indication of the medical condition comprises at least one result chosen from probabilities of an anomaly for different parts of a medical image comprised in the medical data and a numerical value describing a probability of an anomaly of the overall medical data, wherein the numerical value is optionally derived from the probabilities of the anomaly for the different parts of the medical image.

8. The method of any of claims 1 to 7, further comprising determining that a reliable determination of the indication is impossible, if the at least one property associated with the medical data of the test instance does not indicate suitability of the at least one model.

9. The method of any of claims 1 to 8, wherein at least one of the models comprised in the plurality of models (BLS 1 - BLS k) correlates parts of a medical image comprised in the medical data with parts of a reference image, and compares an image value of at least one part of the medical image with information associated with a correlated part of the reference image to obtain the model-specific indication of the medical condition,
wherein the information has been generated by:
matching a plurality of training images to a base image to correlate parts of each of the training images with parts of the base image;
determining image values of at least one part of each of the plurality of training images correlated with a part of the base image, wherein the part of the base image is assigned to the correlated part of the reference image using a predetermined transformation; and
determining the information based on the determined image values of the at least one part of each of the plurality of training images.

10. The method of claim 9, wherein the information comprises or is a statistical distribution function of image values of the at least one part of the plurality of training images.

11. The method of claim 9 or 10, wherein the information comprises an average image value of the at least one part of all of the plurality of training images and, optionally, a mean deviation of the image values of the at least one part of all of the plurality of training images from the average image value.

12. A medical data processing method for determining an indication of a medical condition, the method comprising:
correlating (902) parts of a medical image comprised in medical data of a test instance with parts of a reference image; and
comparing (904) an image value of at least one part of the medical image with information associated with a correlated part of the reference image to obtain the indication of the medical condition,
wherein the information has been generated by:
matching a plurality of training images to a base image to correlate parts of each of the training images with parts of the base image;
determining image values of at least one part of each of the plurality of training images correlated with a part of the base image, wherein the part of the base image is assigned to the correlated part of the reference image using a predetermined transformation; and
determining the information based on the determined image values of the at least one part of each of the plurality of training images, wherein the information is a statistical distribution function of image values of the at least one part of the plurality of training images.

13. An apparatus (100) comprising at least one processor (102) and at least one memory (104), the at least one memory (104) containing instructions executable by the at least one processor (102) such that the apparatus (100) unit is operable to perform the method of any one of claims 1 to 12.

14. A computer program product comprising program code portions for performing the method of any one of claims 1 to 12 when the computer program product is executed on one or more processors.

15. The computer program product of claim 14, stored on one or more computer readable recording media.

## Amended claims

### Amended claims in accordance with Rule 137(2) EPC.

1. A medical data processing method for determining an indication of a medical condition, the method comprising:
selecting (202), based on at least one property associated with medical data of a test instance, at least two models out of a plurality of models (BLS 1 - BLS k), wherein each of the plurality of models (BLS 1 - BLS k) is generated by a learning algorithm and configured to provide a model-specific indication of the medical condition based on the medical data;
determining (204), using each of the selected models, a respective model-specific indication of the medical condition based on the medical data; and
determining (206), based on the model-specific indications, the indication of the medical condition.

2. The method of claim 1, wherein the at least one property associated with the medical data comprises a feature of a medical image comprised in the medical data.

3. The method of claim 1 or 2, wherein the at least one property associated with the medical data comprises a characteristic of a patient to which the medical data relates.

4. The method of any of claims 1 to 3, wherein the step of selecting comprises comparing, individually for each of the plurality of models (BLS 1 - BLS k), the at least one property associated with the medical data of the test instance and at least one property associated with training data used for generating the individual model.

5. The method of claim 4, wherein the indication is determined further based on at least one attribute chosen from a result of the comparing, empirical performances of each of the plurality of models (BLS 1 - BLS k) and a degree of explainability of each of the plurality of models.

6. The method of any of claims 1 to 5, wherein at least one of the models comprised in the plurality of models (BLS 1 - BLS k) is generated by an unsupervised learning algorithm using unlabeled training data of healthy patients and, optionally, configured to provide an anomaly detection as the model-specific indication of the medical condition.

7. The method of any of claims 1 to 6, wherein the model-specific indication of the medical condition and/or the indication of the medical condition comprises at least one result chosen from probabilities of an anomaly for different parts of a medical image comprised in the medical data and a numerical value describing a probability of an anomaly of the overall medical data, wherein the numerical value is optionally derived from the probabilities of the anomaly for the different parts of the medical image.

8. The method of any of claims 1 to 7, further comprising determining that a reliable determination of the indication is impossible, if the at least one property associated with the medical data of the test instance does not indicate suitability of the at least two models.

9. The method of any of claims 1 to 8, wherein at least one of the models comprised in the plurality of models (BLS 1 - BLS k) correlates parts of a medical image comprised in the medical data with parts of a reference image, and compares an image value of at least one part of the medical image with information associated with a correlated part of the reference image to obtain the model-specific indication of the medical condition,
wherein the information has been generated by:
matching a plurality of training images to a base image to correlate parts of each of the training images with parts of the base image;
determining image values of at least one part of each of the plurality of training images correlated with a part of the base image, wherein the part of the base image is assigned to the correlated part of the reference image using a predetermined transformation; and
determining the information based on the determined image values of the at least one part of each of the plurality of training images.

10. The method of claim 9, wherein the information comprises or is a statistical distribution function of image values of the at least one part of the plurality of training images.

11. The method of claim 9 or 10, wherein the information comprises an average image value of the at least one part of all of the plurality of training images and, optionally, a mean deviation of the image values of the at least one part of all of the plurality of training images from the average image value.

12. A medical data processing method for determining an indication of a medical condition, the method comprising:
correlating (902) parts of a medical image comprised in medical data of a test instance with parts of a reference image; and
comparing (904) an image value of at least one part of the medical image with information associated with a correlated part of the reference image to obtain the indication of the medical condition,
wherein the information has been generated by:
matching a plurality of training images to a base image to correlate parts of each of the training images with parts of the base image;
determining image values of at least one part of each of the plurality of training images correlated with a part of the base image, wherein the part of the base image is assigned to the correlated part of the reference image using a predetermined transformation; and
determining the information based on the determined image values of the at least one part of each of the plurality of training images, wherein the information is a statistical distribution function of image values of the at least one part of the plurality of training images.

13. An apparatus (100) comprising at least one processor (102) and at least one memory (104), the at least one memory (104) containing instructions executable by the at least one processor (102) such that the apparatus (100) unit is operable to perform the method of any one of claims 1 to 12.

14. A computer program product comprising program code portions for performing the method of any one of claims 1 to 12 when the computer program product is executed on one or more processors.

15. The computer program product of claim 14, stored on one or more computer readable recording media.
